# EUROPEAN PATENT APPLICATION

(11) **EP 2 149 344 A1**
(43) Date of publication of application: **03.02.2010**
(21) Application number: 09009928.4
(22) Date of filing: 31.07.2009
(51) Int. Cl.: A61B 18/24, A61N 5/067, A61B 18/22, A61N 5/06, A61B 17/00

(54) **Devices for the treatment of BPH and for ablation of tissue**

(30) Priority: 01.08.2008 US 184910
(71) Applicant: Dornier MedTech Laser GmbH, 82234 Wessling (DE)
(72) Inventor: Rheinwald, Markus, Dr., 86916 Kaufering (DE); Hiereth, Werner, 82205 Gilching (DE)
(74) Representative: Grünecker, Kinkeldey, Stockmair & Schwanhäusser Anwaltssozietät

(57) **Abstract**

The invention refers to a device for the treatment of Benign Prostatic Hyperplasia, comprising a laser diode module (7) configured to generate laser radiation with a peak wave length in the range of 920 nm to 960 nm and an average output power of more than 200 W; and an optical application fiber (10) configured to guide the laser radiation generated with the laser diode module (7) to human prostate tissue (19) such that the laser radiation has a spot size at the human prostate tissue of between 0.8 mm2 and 1.5 mm2 and a penetration depth in the human prostate tissue of 3 mm to 6 mm, wherein the device is configured to create a thermally damaged layer in the human prostate tissue with a thickness between 3.5 mm to 6 mm, and wherein the device operates in a continuous wave mode. Further the invention refers to a device for ablation of tissue, preferably human prostate tissue with laser radiation, the device including a laser radiation generator having laser diodes for generating the laser radiation with a wave length between 800 nm to 1000 nm; a connector (9) for connecting an optical application fiber (10) for delivering the laser radiation to the tissue (19), and the device having an average output power of the laser radiation of more than 150W.

## Description

### TECHNICAL FIELD

Devices for the treatment of Benign Prostatic Hyperplasia ("BPH") and for ablation of tissue.

### BACKGROUND

BPH is an enlargement of the human prostate that occurs frequently for elderly men. U.S. Patent Publication No. 2003/0135205 to Davenport et al. ("Davenport") discloses treating BPH by generating laser radiation with an Nd:YAG laser at 1064 nm. The laser is frequency doubled such that a wavelength of 532 nm is obtained. To obtain a reasonable frequency doubling efficiency, the device has to be operated in a pulse mode. In this way, the laser can achieve the high peak intensities which lead to a reasonable frequency doubling efficiency.

The wavelength of 532 nm has a high absorption rate in hemoglobin, which is present in the tissue, resulting in the penetration depth being as small as 0.8 mm. This leads to a high vaporization rate due to the concentration of laser radiation in the surface of the treated tissue. In this Davenport, this is compared with a wavelength of 1064 nm where hardly any vaporization is found at a high penetration depth. This is highly undesired because it causes tissue to be thermally damaged but not removed.

German Patent Application Publication DE 10 2006 037289 A1 discloses a device for vaporization of tissue by means of diode laser radiation. Specifically, it discloses emitting the laser light in a pulse mode or in pulse packets in a variable pulse pause ratio. The variable pulse configuration is described to be advantageous in order to avoid an undesired carbonization of the irradiated tissue. This is due to purposely lowering the temperature of the tissue between the radiation pulses but, at the same time, providing a high vaporization quality during the pulse.

A scientific publication by Wendt-Nordahl, G. et al., entitled "980 nm Diode Laser: A Novel Laser Technology for Vaporization of the Prostate", Eur Urol 2007, discloses that, in ex vivo porcine kidneys, the coagulation zone depth in a continuous wave ("CW") mode is significantly larger than in a pulse mode.

Additionally, a publication by M. Seitz, et al., entitled "Der Dioden Laser, Ex Vivo - Untersuchungen zu den Vaporisations- und Koagulationseigenschalten", Urologe A 2007, 46: 1242 - 1247, discloses that for a 980 nm laser, the coagulation depth increases from 5.7 mm to an unacceptable 10.2 mm when increasing laser radiation at a wavelength of 980 nm from 30 to 100 Watt.

When trying to reduce the treatment time of a patient, it may be considered to try to increase the power of the laser system as disclosed in Davenport. However, this approach is undesirable because it increases the probability of bleeding after treatment. This was found out to be due to an explosion-like vaporization of the surface tissue which mechanically affects deeper lying tissue such that this tissue starts to bleed. This is highly undesirable since such bleeding usually causes complications after the treatment of a patient.

Due to the wavelength of 532 nm, which is strongly absorbed in particular in hemoglobin, (present in blood), tissue with a high blood content is vaporized and mechanically destructed due to the explosion-like vaporization. This effect additionally leads to the undesired bleeding.

### SUMMARY

The object of the invention is therefore to provide a laser system which, on the one hand allows for a reduced treatment time of patients as compared to the prior art and, at the same time, avoids undesired bleeding with a high probability.

The object of the invention is met by a device for the treatment of BPH including a laser diode module for generating laser radiation with a peak wave length in the range of 920 to 960 nm and an average output power of more than 200 W. The device also includes an optical application fiber for guiding the laser radiation generated with the laser diode module to human prostate tissue. The device operates such that a spot size of laser radiation at the human prostate tissue between 0.8 mm² and 1.5 mm² is obtained; and a penetration depth of the laser radiation in the human prostate tissue of 3 to 6 mm is obtained. Further, the device is capable of creating a thermally damaged layer with a thickness between 3.5 mm to 6 mm, and the device operates in a continuous wave mode.

The invention also includes a method of treating BPH, wherein laser radiation is applied to human prostate tissue with an endoscopic laser radiation guiding fiber. The laser radiation has a power of at least 200 W and a peak wave length in the range of 920 to 960 nm, and is applied in a continuous wave mode. The laser radiation on a surface of the human prostate tissue has a spot size between 0.8 mm² and 1.5 mm²; a penetration depth in the human prostate tissue having a range of 3 mm to 6 mm; and a layer of thermally damaged tissue is created with a thickness having a range of 3.5 mm to 6 mm.

The invention also includes a method of treating BPH wherein laser radiation is applied to prostate tissue with an endoscopic laser radiation guiding fiber such that, a surface region of the prostate tissue is vaporized; and in a region further away from the endoscopic laser radiation guiding fiber than the surface region, the tissue is thermally damaged. Further, a three dimensional vaporization rate is at least one of: more than 5 mm³/s; more than 10 mm³/s; more than 20 mm³/s; more than 30 mm³/s; more than 40 mm³/s; more than 50 mm³/s; more than 75 mm³/s; and more than 100 mm³/s; and the average laser power is more than 100 W.

The invention also includes a device for ablation of tissue with laser radiation which includes a laser radiation generator having laser diodes for generating the laser radiation with a wave length between 800 nm to 1000 nm. The device also includes a connector for connecting an optical application fiber for delivering the laser radiation to the tissue; and has an average output power of the laser radiation of more than 150W.

In an embodiment of the invention BPH can be treated with laser radiation which has a wave length between 920 and 960 nm such as 940 nm. In this wavelength range, in comparison to the wavelength of 532 nm, absorption in water is approximately a factor of 100 higher. Absorption in hemoglobin, however, is approximately a factor of 50 lower in comparison to that of the wavelength of 532 nm.

In particular, due to the reduced absorption in hemoglobin, the penetration depth of light at this wavelength is higher. It was found that, nevertheless, good vaporization rates could be achieved for an output power of more than 150 Watt.

It was found that the zone wherein tissue becomes thermally damaged but does not vaporize reduces upon an increase of the average laser power. In this thermally damaged zone, coagulation takes place, however. The coagulation is desirable, at least within a certain thickness, in order to avoid the bleeding of the tissue. The coagulated tissue seals the blood vessels and therefore is capable of avoiding bleeding of the tissue which is vaporized due to the laser irradiation. It was also found that this thermally damaged zone in which coagulation takes place may be controlled to have a value between 3.5 and 6 mm. A value of 3.5 mm is sufficient to avoid bleeding and a value of 6 mm is sufficiently small in order to not destroy too much tissue without vaporizing it.

Furthermore, contrary to certain literature published, it has been found out that a continuous wave mode is advantageous in comparison to pulse modes. In the continuous wave mode, the average laser power is the highest for a given maximum output power. This is particularly useful for obtaining a high average power which, as explained above, can be used to obtain a thickness of the thermally damaged zone in the desired range of 3.5 and 6 mm. Therefore, laser radiation with a continuous wave is preferred. In particular, for average laser powers of more than 150 W the thickness of the thermally damaged zone can be in a preferred range between 3.5 and 6 mm.

The device is provided with an optical application fiber. This fiber may be inserted into an endoscope in order to provide the laser radiation towards the tissue to be treated. The spot size of the laser radiation is preferably in the range of 0.8 mm² to 1.5 mm². This leads, on the one hand, to a spot size sufficiently small in order to have the laser radiation concentrated enough to provide vaporization. But, on the other hand, it leads to the radiation covering an area which is sufficiently large in order to provide short treatment times, since a big amount of volume of tissue can be treated at the same time.

A large amount of tissue which can be treated at the same time is also achieved by a penetration depth into the tissue of 3 to 6 mm. The penetration depth is defined by the distance from the tissue surface at which the laser radiation intensity has dropped to 1/e of the initial value.

The output power may be more than 200, 250, 300, 500, 1000 or 2000 Watt as well. Since the penetration depth of the light is a few millimeters, the energy coupled into the tissue does not lead to an explosion-like vaporization but to a more controlled vaporization which can be carried out at significantly higher powers.

With the laser device, the laser irradiation can be applied to a volume of tissue. When assuming that the treated tissue essentially corresponds to a volume which is given by the spot size multiplied by the penetration depth, the three dimensional laser power density was found to be preferably less than 500 kW/cm³. More preferable is a three dimensional laser power density of less than 100 kW/cm³. It is estimated that, at a three dimensional laser power density of 500 kW/cm³, the above-mentioned mechanical destruction of tissue sets in which leads to the undesired bleeding. In order to be in a range where this mechanical destruction of tissue does not appear, it is preferred to stay below 500 kW/cm³ or even below 100 kW/cm³.

Nevertheless, in order to have sufficient vaporization which allows for short treatment times, a three dimensional laser power density of more than 50 kW/cm³ is desired. It is estimated, that vaporization sets in at a three dimensional laser power density of approximately 40kW/cm³.

Due to the relatively high penetration depth, more than 1 or even up to more than 20 mm³ of human prostate tissue is treatable at the same time. The treatment refers to the volume of tissue within which the laser radiation intensity is more than 1/e of the maximum intensity at a specific location with the maximum intensity.

The device is preferably adapted to have a three dimensional tissue ablation rate of more than 5mm³/sec or even up to more than 50 or 100 mm³/sec. This leads to significantly reduced treatment times. If, for example, 10g of tissue is to be removed, at a three dimensional tissue ablation rate of 100 mm³/sec, this will last only 100 seconds.

In a method for treating BPH, laser radiation is applied to the prostate tissue with an endoscopic laser radiation guiding fiber. In the surface region, the prostate tissue is vaporized and in a region further away from the laser radiation guiding fiber than the surface region, the tissue is thermally damaged. The three dimensional vaporization rate, however, is more than 5 mm³/sec or even up to more than 100 mm³/sec. The laser power in this case should be higher than 100 W. It can be even as high as 2 kW.

The laser radiation preferably has a peak wavelength between 800 nm and 1000 nm. In this range high tissue vaporization rates can be achieved due to the relatively high penetration depth of a few millimeters, which leads to a large volume of tissue which can be treated at the same time. Preferred is a wavelength of more than 850, 900 or 920 or 930 nm and/or less than 990 nm, 980 nm, 970 nm, 960 nm or 950 nm.

The laser radiation is preferably continuous wave or with a ratio of the pulse length to the cycle length of more than 0.9 or 0.95. The ratio of the average power to the maximum power during treatment is preferably more than 0.9 or 0.95.

The laser irradiation is irradiated towards the tissue with the spot size on the surface of the tissue of less than 10 mm². Preferred is a spot size with a surface of more than 0.8 mm². In a specific embodiment the spot size is around 1.0 mm² or 1.5 mm².

By varying the wavelength (preferably within the wavelength range of 800 nm to 1000nm) the penetration depth can be controlled. Here a penetration depth of more than 2 mm and/or less than 6 mm is preferred.

In a particular embodiment for ablation of tissue, preferably human prostate tissue with laser radiation, the device emits laser radiation of a diode laser system between 800 nm and 1000 nm. The device has a connector for connecting an optical application fiber. With this fiber the radiation can be applied to tissue. The average output power is preferably more than 150 W in order to have thermally damaged zone of not more than 6 mm. Any of the above or below mentioned options apply to this embodiment as well.

The tissue may be any living tissue, preferably human tissue, even more preferably any tissue that forms high volume organs or pathologic entities, even more preferably tissue that is well perfused with blood. This can be, for example, but not limited to, any benign or malignant hyperplastic tissue such as myoma of any host organ, or brain tumors; but also organ masses with other pathologies causing a need for them to be vaporized, preferably parenchymal organs, e.g. kidney or liver or others.

The average laser power may for example be up to more than 200 W or even more than 2 kW. Preferred is a wavelength of more than 850 nm, 900 nm or 920 nm or 930 nm and/or less than 990 nm, 980 nm, 970 nm, 960 nm or 950 nm and this preferably in a CW mode.

The fiber may be a bare fiber or a side-firing fiber. In general, a side-firing fiber is preferred.

With the above mentioned devices and methods it is possible to treat patients of BPH within a relatively short time in comparison to the prior art and, at the same time, avoiding bleeding or excessive thermal damage more than necessary.

### BRIEF DESCRIPTION OF THE DRAWINGS

Figure 1 shows a schematic view of a laser device, in accordance with an exemplary embodiment of the invention.
Figure 2a shows a cross-section of a fiber in the tissue, in accordance with an exemplary embodiment of the invention.
Figure 2b shows different zones of treated tissue, in accordance with an exemplary embodiment of the invention.
Figure 3 shows a cross-section of a dog prostate after treatment wherein the different zones are indicated, in accordance with an exemplary embodiment of the invention.
Figure 4 shows a diagram relating the thickness of the coagulated zone d in dependence of the average laser power, in accordance with an exemplary embodiment of the invention.

### DETAILED DESCRIPTION

Exemplary embodiments of the invention are provided. These embodiments include a device for the treatment of BPH having: a laser diode module for generating laser radiation with a peak wave length in the range of 920 to 960 nm and an average output power of more than 200 W, wherein the device operates in a continuous wave mode; and an optical application fiber for guiding the laser radiation generated with the laser diode module to human prostate tissue such that, a spot size of laser radiation at the human prostate tissue between 0.8 mm² and 1.5 mm² is obtained; and a penetration depth of the laser radiation in the human prostate tissue of 3 to 6 mm is obtained; wherein the device is capable of creating a thermally damaged layer with a thickness between 3.5 mm to 6 mm. The invention also includes to a corresponding method with a high three dimensional vaporization rate and a device for ablating tissue.

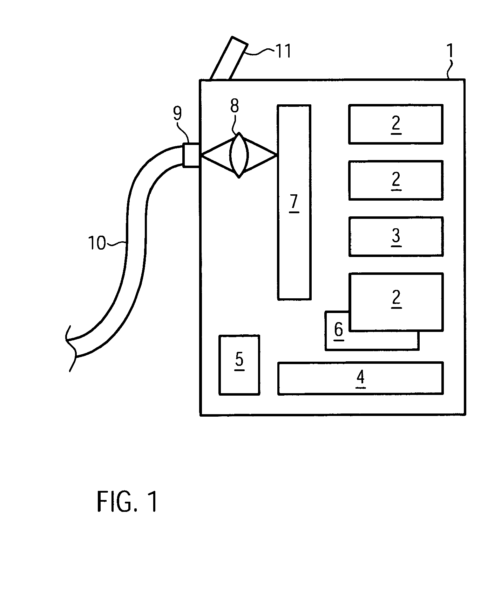

In Figure 1, a device 1 with a laser diode module 7 is shown. Power supply modules of the laser diode module 7 are shown as items 2. Item 3 refers to a central processing unit. Item 4 is an electronic component for the current supply of the device 1. Item 6 is a heat exchanger and item 5 is a container for cooling liquid. The device 1 itself does not need an external liquid cooling such as a water cooler but can be air cooled. Air ventilated into the device 1 cools the cooling liquid through interaction in the heat exchanger 6. Item 11 is a screen and some control means with which the operation of the device can be controlled by a user.

The laser diode module 7 irradiates laser radiation which is coupled with an optical means 8 into a connector port 9 of the device. In Figure 1, fiber 10 for guiding laser radiation is shown. This fiber may have a light guiding core with a diameter of 400 µm, 500 µm, 600 µm or more.

The laser diode module 7 comprises numerous laser diodes, the irradiation of which is coupled together to form the beam which can be coupled into the fiber 10.

The laser radiation has a wavelength of 940 ±5, 10 or 20 nm, but other wavelengths in the range of 800 nm - 1000 nm may be possible as well. The following explanations for a wavelength of 940 nm are an example of the wavelengths of 800 nm - 1000 nm.

The laser module 7 is capable of outputting a laser power of up to 250 Watt in a continuous wave mode. The output power may, however, be reduced by operation of the central processing unit 3.

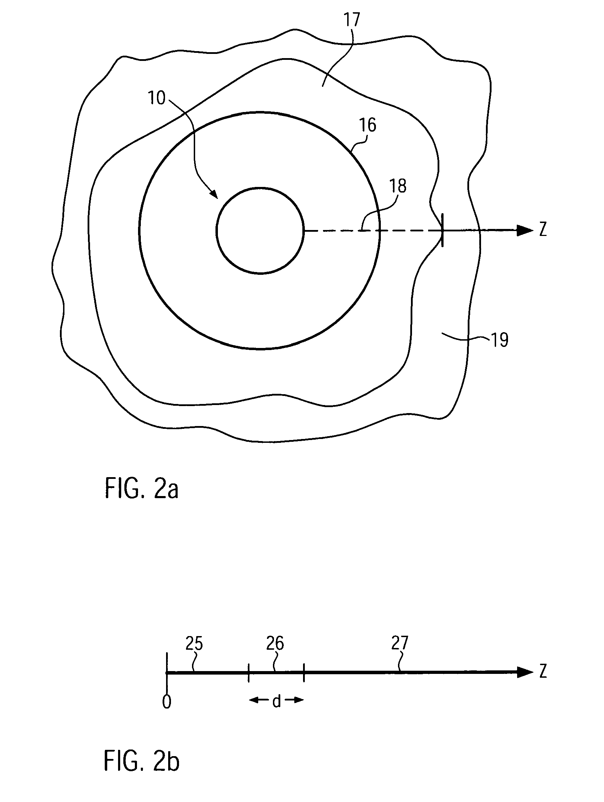

In Figure 2a, a cross-section through the fiber 10 is shown. The fiber 10 is provided through an endoscopic device 16. The endoscopic device 16 can provide a liquid 17 to the application zone of the laser radiation in order to clean the zone. The liquid 17 may also be removed through the endoscope 16 in order to remove ablated tissue remains. The fiber 10 sticks out of the end of the endoscope 16 such that the laser radiation can be applied to the tissue in front of the end of the endoscope 16. In Figure 2a, a side firing fiber is shown which emits the radiation to the side of the longitudinal axis of the endoscope. Around the end of the fiber 10 which sticks out of the endoscope 16, there is provided tissue 19 to be treated. Between the fiber 10 and the tissue 19 there is a liquid 17 which has been inserted through the endoscope 16 in order to create a cavity in which the end of the endoscope 16 can me moved. Thus allowing the fiber 10 to be positioned in the desired area. In Figure 2a, an axis z that is shown which has its zero point at the surface of the tissue 19.

After treatment, for example, of prostate tissue, along the direction z shown in Figure 2a, different zones may be identified as shown in Figure 2b. Close to the initial surface, a zone 25 of vaporized tissue where the tissue has been removed can be found. This zone is called surface zone, since initially it lies next to the surface of the tissue. Between the zones 25 and 26, there may be some carbonized tissue. In the zone 26, which has a thickness d, the tissue is thermally damaged i.e. coagulated. In zone 27, the tissue is unaltered.

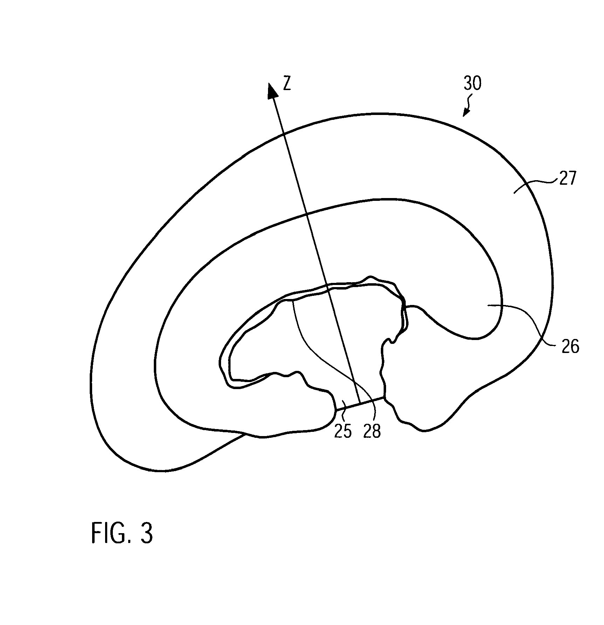

In Figure 3, an example of a cross-section 30 of a dog prostate is shown after treatment with laser radiation at 940 nm and 200 Watt CW. For easier orientation, the z-axis of Figures 2a and 2b is shown. (Surface) zone 25 is free of tissue and shows where tissue has been removed. In the zone 28, some tissue remainders of the vaporized tissue or some slightly carbonized tissue can be found. In zone 26, coagulated (thermally damaged) tissue is identified. The zone 27 refers to unaltered tissue. The thickness d of the layer 26 is in the range of 2-6 mm.

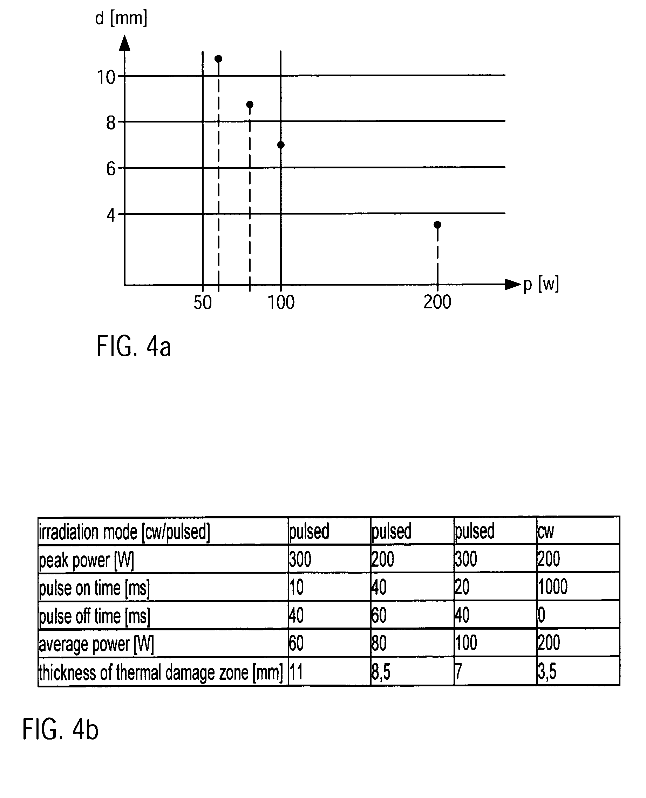

Figure 4a shows a diagram which relates the average power in Watt to the thermal damage or coagulation zone thickness d. The data on which the measurements shown in Figure 4a are based is shown in the table in Figure 4b. As can be seen, in comparison to the pulse modes in the continuous 200 Watt mode, a significantly reduced thickness of the thermally damaged zone can be achieved in CW mode.

In a study, five dog prostates were treated in vivo for a time of 30 seconds at an output power of 200 Watt continuous wave mode at 940 nm. The total applied energy was 6000 Joule. The ablation depth was found to be between 5.5 mm and 6 mm and this over an ablation length of approximately 15 mm (the length is measured along the fiber length). In total, a volume of approximately 600 - 800 mm³ was ablated. The coagulation depth was found to be approximately 4 mm which is sufficient to prevent bleeding and not to destroy too much prostate tissue without ablating it.

Yet another embodiment of the invention includes a device for ablation of tissue with laser radiation. The device includes a laser radiation generator having laser diodes for generating the laser radiation with a wave length between 800 nm to 1000 nm; and a connector for connecting an optical application fiber for delivering the laser radiation to the tissue. The device has an average output power of the laser radiation of more than 150W.

In this embodiment, the average output power of the laser radiation can be greater than 200 W and/or greater than 2 kW. The peak wavelength is in a range with a minimum value of one of: 850 nm; 900 nm; 920nm; and 930 nm; and a maximum value of one of: 980 nm; 960 nm; and 950 nm. The laser radiation can be continuous wave or in pulse mode having a ratio of the pulse length to the cycle length of more than 0.9 or 0.95. An optical application fiber is connected to the device with which the laser radiation can be irradiated towards the tissue with a spot size of less than 10 mm² and/or greater than 0.5 mm². The optical application fiber is connected to the device with which the laser radiation can be irradiated towards the tissue with a three dimensional laser power density of less 500 kW/cm³ and/or more than 50 kW/cm³. The laser radiation has a penetration depth in the tissue that is greater than 2 mm and/or less than 6 mm. The volume of simultaneously treatable tissue is greater than 1 mm³ and/or less than 30 mm³. Further, the device yields a three dimensional tissue ablation rate of greater than 5 mm³/s and/or greater than 100 mm³/s. The device is further capable of creating a thermally damaged layer with a thickness between 2 mm and 6 mm.

## Claims

1. A device for the treatment of Benign Prostatic Hyperplasia, comprising:
a laser diode module (7) configured to generate laser radiation with a peak wave length in the range of 920 nm to 960 nm and an average output power of more than 200 W; and
an optical application fiber (10) configured to guide the laser radiation generated with the laser diode module (7) to human prostate tissue (19) such that the laser radiation has a spot size at the human prostate tissue of between 0.8 mm² and 1.5 mm² and a penetration depth in the human prostate tissue of 3 mm to 6 mm,
wherein the device is configured to create a thermally damaged layer in the human prostate tissue with a thickness between 3.5 mm to 6 mm, and wherein the device operates in a continuous wave mode.

2. The device of claim 1, wherein the average output power of the laser radiation is greater than 250 W, 300 W, 500 W, 1 kW, or 2 kW.

3. The device according to claim 1 or 2, wherein the device is configured to irradiate the laser radiation towards the human prostate tissue (19) with a three dimensional laser power density of less than 500 kW/cm³ or 400 kW/cm³ or 300 kW/cm³ or 200 kW/cm³ or 100 kW/cm³ and/or more than 50 kW/cm³ or 70 kW/cm³.

4. The device according to any of claims 1 to 3, wherein a volume of simultaneously treatable human prostate tissue is more than 1 mm³ or 3 mm³ or 5 mm³ or 10 mm³ or 20 mm³ and/or less than 40 mm³ or 30 mm³ or 20 mm³ or 10 mm³ or 5 mm³.

5. The device according to any of claims 1 to 4, wherein the device is configured to achieve a three dimensional tissue ablation rate of more than 5 mm³/s or 10 mm³/s or 20 mm³/s or 30 mm³/s or 40 mm³/s or 50 mm³/s or 75 mm³/s or 100 mm³/s.

6. Device for ablation of tissue, preferably human prostate tissue with laser radiation, the device including:
a laser radiation generator having laser diodes for generating the laser radiation with a wave length between 800 nm to 1000 nm;
a connector (9) for connecting an optical application fiber (10) for delivering the laser radiation to the tissue (19), and
the device having an average output power of the laser radiation of more than 150W.

7. The device of claim 6, wherein the average output power of the laser radiation can be greater than 200 W, 250 W, 300 W, 500 W, 1 kW or 2 kW.

8. The device of any of claims 6 or 7, wherein the peak wavelength is in a range with a minimum value of one of: 850 nm, 900 nm, 920 nm, and 930 nm and/or a maximum value of one of: 980 nm, 960 nm, and 950 nm.

9. The device of any of claims 6 to 8, wherein the laser radiation can be continuous wave or in pulse mode having a ratio of the pulse length to the cycle length of more than 0.9 or 0.95.

10. The device of any of claim 6 to 9, wherein an optical application fiber is connected to the device with which the laser radiation can be irradiated towards the tissue (19) with a spot size of less than 10 mm² or 8 mm² or 6 mm² or 4 mm² or 3 mm² or 2 mm² or 1 mm² and/or greater than 0.5 mm² or 0.7 mm² or 0.8 mm².

11. The device of any of claims 6 to 10, wherein the optical application fiber (10) is connected to the device (1) with which the laser radiation can be irradiated towards the tissue with a three dimensional laser power density of less 500 kW/cm³ or 400 kW/cm³ or 300 kW/cm³ or 200 kW/cm³ or 100 kW/cm³ and/or more than 50 kW/cm³ or 70 kW/cm³_{.}

12. The device of any of claims 6 to 11, wherein the laser radiation has a penetration depth in the tissue that is greater than 2 mm or 3 mm or 4 mm or 5 mm and/or less than 6 mm or 5 mm or 4 mm.

13. The device of any of claims 6 to 12, wherein the volume of simultaneously treatable tissue is greater than 1 mm³ or 3 mm³ or 5 mm³ or 10 mm³ or 20 mm³ and/or less than 40 mm³ or 30 mm³ or 20 mm³ or 10 mm³ or 5 mm³.

14. The device of any of claims 6 to 13, wherein the device yields a three dimensional tissue ablation rate of greater than 5 mm³/s or 10 mm³/s or 20 mm³/s or 30 mm³/s or 40 mm³/s or 50 mm³/s or 75 mm³/s or 100 mm³/s.

15. The device of any of claims 6 to 14, wherein the device is further capable of creating a thermally damaged layer with a thickness between 2 mm and 6 mm.
